# EUROPEAN PATENT APPLICATION

(11) **EP 1 161 967 A1**
(43) Date of publication of application: **12.12.2001**
(21) Application number: 00306596.8
(22) Date of filing: 02.08.2000
(51) Int. Cl.: A61N 2/02, A61N 2/06

(54) **Electromedical treatment applicator**

(30) Priority: 08.06.2000 GB 0014016
(71) Applicant: Med-Tech A/S, 9881 Bindslev (DK)
(72) Inventor: Lindholt, Claus, 9700 Broenderslev (DK)
(74) Representative: Cross, Rupert Edward Blount

(57) **Abstract**

A device for electromedically treating body parts by inducing movement in electrically charged particles in the body part is disclosed. The device comprises a pair of solenoid coils (20a, 20b) which are electrically connected together by a wire 25. A first permanent magnet (30a) is mounted within the bore of the first solenoid coil (20a), and a second permanent magnet (30b) is mounted within the bore of the second solenoid coil (20b). Each solenoid coil (20a, 20b) is connected to a power supply (130). The device is thus much more compact than previous devices which required large heavy power supplies to generate the magnetic fields by an electromagnet, and also to generate electric fields as well. In the preferred embodiment, the device is handheld.

## Description

This invention relates to an electromedical treatment apparatus for the treatment of biological tissue, bone and so forth.

Electromagnetic fields have for some time been employed in the treatment of many parts of the body. The interaction of the electromagnetic field with charged particles has been shown to improve the rate of healing of, for example, broken bones and torn ligaments, impaired blood circulation due to arteriosclerosis and the like, and also appears to have a palliative effect on rheumatism and other chronic ailments.

Blood contains a number of different ionic particles such as potassium, sodium, calcium, iron, chloride, sulphate and phosphate ions. As these ions pass through an electromagnetic field, the ions are caused to move relative to their original direction of motion as a consequence of the Lorentz force thereon. This in turn causes an increase in blood flow in the region of the electromagnetic field. The localized oversupply of blood, together with an increased throughflow, is known as hyperaemia and it is this which increases the rate of healing. The increased blood flow assists the transportation of nutrients, protein and oxygen to the damaged tissue or bone cells; the movement of charged particles also causes uncharged particles to be disturbed because of the relative viscosity of the blood.

A device for generating a 'crossed' electromagnetic field for electromedical treatment is described in EP-A-0,062,032. The device comprises two coaxially arranged annular coils which together generate a magnetic field largely confined between the two coils and having field lines that are generally linear in the region between the two coils. The two annuli each include both a magnetic and an electric lens.

In addition, a pair of coaxial electrodes are provided to generate an electrostatic field. The electrodes are arranged between the coils so that the notional central axis of the electrodes intersects the notional central axis of the coils perpendicular thereto and substantially equidistantly from each coil.

A d.c. current is supplied to the windings of the coils to form a bias (constant amplitude) magnetic field. A half-wave rectified sinusoidal signal (which does not pass zero), having a frequency of between 20 and 100 Hz, is also commonly supplied to the electrodes, the coils and the electric and magnetic lenses.

Whilst the device described in EP-A-0,062,032 has proved to be commercially successful, it does suffer from a number of drawbacks.

Firstly, the device is relatively complicated and is therefore costly to manufacture. The complexity also increases the size of the electromagnetic 'head' comprising the pair of coils, the electric and magnetic lenses and the electrodes, particularly since the electrodes need to be located so as to generate an electric field perpendicular to the magnetic field (to prevent z-axis Lorentz forces being generated: **F**=Q(**E**+**VxB**)). Moreover, to generate the various electric and magnetic fields, the power supply typically needs to generate a d.c. current between 1A and 5A. Therefore, it needs to be relatively large and heavy and this in turn further reduces the ease with which the device may be moved around.

It is an object of the present invention to overcome these problems with the prior art.

According to the present invention, there is provided an apparatus for electromedically treating body parts by inducing movement in electrically charged particles in said tissue, comprising: a first coil, to generate a dynamic magnetic field when the coil is supplied with an a.c. signal; and a first permanent magnet structure to generate a static bias magnetic field, the first permanent magnet structure being arranged in proximity to the first coil such that, in use, a composite magnetic field comprising the dynamic magnetic field superimposed upon the static magnetic field is generated.

By generating the magnetic field used to treat biological tissue and the like using a combination of permanent and electromagnets, the resultant device can be dramatically miniaturised yet still generate a suitably large magnetic field for the electromedical treatment of body parts such as injured tissue or bones. Superimposing a dynamic magnetic field onto a quiescent or bias static magnetic field means that the dynamic magnetic field can be generated solely through an a.c. signal. This in turn minimises power losses which were significant in previous devices, and hence allows the power for the coil in the present invention to be supplied, for example, from a battery.

It has also been discovered that the electric and magnetic lenses of EP-A-0,062,032 have minimal effect on the electric and magnetic fields in the treatment zone of that device. Furthermore, the electrodes for generating a separate electric field can be dispensed with as well. Therefore, the device of the present invention can operate without any large power sources being required at all. Current embodiments of the invention are sized to fit into the palm of a hand.

In order further to enhance the magnetic field strength of the dynamic magnetic field generated by the first coil, the first coil may further comprise a soft iron core member. The coil may then be arranged at least partially to surround the core member. Preferably, the core member has a first surface facing towards a body part to be treated when in use, which first surface is concave. This causes the lines of magnetic flux to be focussed towards the body part. Additionally or alternatively, the core member may have a second surface facing away from the body part to be treated when in use, which second surface may be convex in order to minimise the spread of the magnetic field away from the treatment region.

In one embodiment, the first coil may be an annular solenoid having a solenoid bore, the first permanent magnet structure being arranged within the solenoid bore of the first coil. This is a particularly ergonomic design. In an alternative embodiment, however, the first coil may be an annular solenoid having end faces, with the first permanent magnet structure comprising a plurality of permanent magnets arranged radially around the end faces of the coil.

The first permanent magnet structure may be formed of a ceramics material. Ceramic magnets have a very low electrical conductivity. This means that eddy currents and thus eddy current losses will be minimal. It is for this reason that, the permanent magnet structure may be placed inside the solenoid bore of the first coil.

Preferably, the dynamic magnetic field is generated by the application of a sawtooth waveform to the first coils. Although a number of electronic circuits are capable of generating such a waveform, a half bridge configuration is particularly preferred.

Preferably, the ratio of the rise time to the fall time of the sawtooth waveform is between 40:60 and 50:50. These ratios, together with a signal frequency of around 100-1200 Hz (most preferably 600-800 Hz), have been found to follow the natural shape of cell signals. However, it will be understood that other waveforms are envisaged, including square and sinusoidal waves, the latter requiring even less energy for its generation.

In order to allow the magnitude of the static bias magnetic field to be altered, the orientation of the permanent magnet structure is relative to the coils is preferably adjustable.

In view of the magnitude of the composite magnetic field required for treatment, a single coil/permanent magnet structure is possible. Nevertheless, in a preferred embodiment, the apparatus further comprises a second coil also arranged to generate a dynamic magnetic field when supplied with an a.c. signal, the first and second coils being laterally displaceable from one another; and a second permanent magnet structure arranged in proximity to the second coil, wherein the composite magnetic field is generated in a treatment region defined between the two coils when laterally displaced.

The advantageous features of the first coil and/or first permanent magnet structures as set out previously are, of course, also advantageous features of the second coil and/or second permanent magnet structures, mutatis mutandis.

The invention may be put into practice in a number of ways, and embodiments will now be described by way of example only and with reference to the accompanying drawings in which:
Figure 1 shows, schematically, the relative orientation of a blood vessel, electrically charged particles within the blood flowing along it, and the preferred direction of applied magnetic field for electromedical treatment;
Figure 2 shows the resultant movement of a charged particle when a static magnetic field is applied in the direction shown in Figure 1;
Figure 3 shows a schematic plan view of the electromedical treatment apparatus embodying the present invention;
Figure 4 shows the apparatus of Figure 3 in schematic perspective view;
Figure 5 shows a waveform suitable for application to the coils of Figures 3 and 4;
Figure 6 shows schematically a circuit suitable for generating the waveform of Figure 5;
Figure 7 shows schematically an alternative circuit suitable for generating the waveform of Figure 5.
Figures 8a and 8b respectively show sectional and plan views of an alternative embodiment of an electromedical treatment apparatus in accordance with the present invention; and
Figure 9 shows a schematic perspective view of the apparatus of Figures 3 to 8, mounted within a housing together with a power supply.

Before explaining the structure of the electromedical apparatus embodying the present invention, a brief discussion of the biophysics underlying the use of electromagnetism in medical treatment of body parts will first be provided. However, it is to be understood that the medical and biological effects caused by this apparatus do not of themselves form a part of the present invention and are therefore discussed briefly to allow a better understanding of the apparatus itself. The use of electromagnetism in the treatment of injuries is a complex subject which is not yet fully understood. A more detailed explanation of this subject is given in, for example, the above-referenced European Patent Application, EP-A-0,062,032.

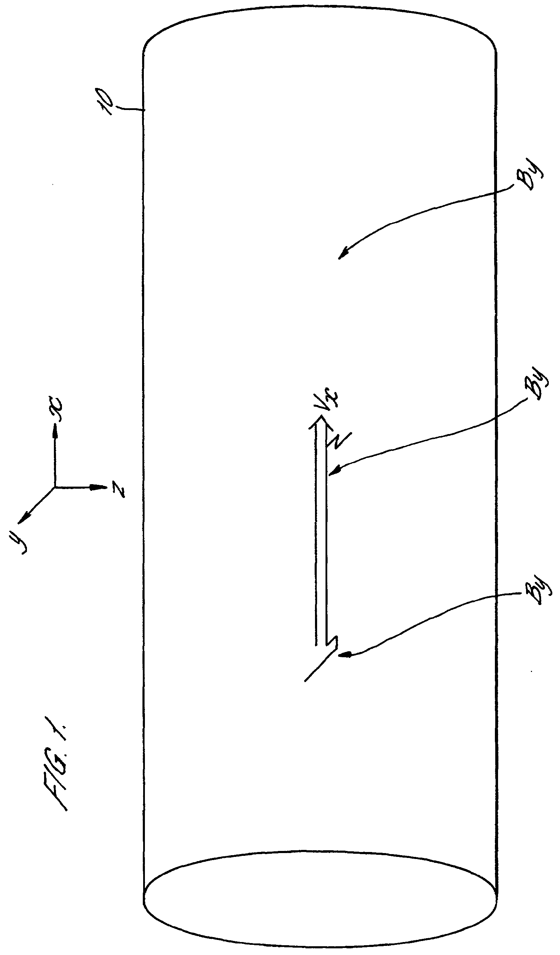

Referring first to Figure 1, the relative orientation of a blood vessel, electrically charged particles within blood flowing along it, and the preferred direction of applied magnetic field for electromedical treatment is shown schematically. Charged particles move along a blood vessel 10, in the example of Figure 1 with a velocity component in the x-direction only. An applied magnetic field B has a component in the y-direction only, and thus the charged particles in the blood vessel 10 travel at right-angles to the direction of applied magnetic field.

Assuming no electric field is present, the Lorentz force upon the charged particles will be in the z-direction. In fact, some of the particles may move in the z-direction, depending upon their polarity. Provided that the speed of the charged particle in the magnetic field is constant, and that the magnetic field itself is constant in time and is homogeneous, then the charged particles will move in a circular path.

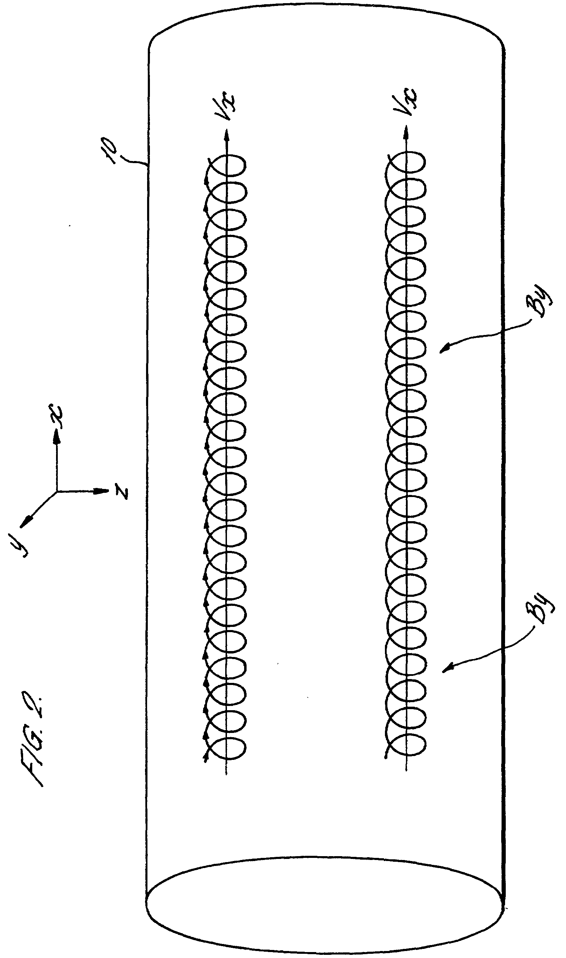

Combining the Lorentz force on the charged particles with the force on them due to blood pressure, a spiral trajectory as shown in Figure 2 is produced. Of course, in reality, the applied magnetic field will not be entirely homogeneous. Furthermore, it is possible to add a time-varying component to the magnetic field. All of this causes the charged particles to describe a distorted spiral around the net direction of flow.

The consequence of this is that, relative to particles travelling in a field-free zone, the charged particles here have an equivalent mean-free path before collision which is shorter, that is, they undergo more collisions per unit length in the axial direction. It is this decreased equivalent mean-free path which is a major factor in the production of a medically beneficial effect.

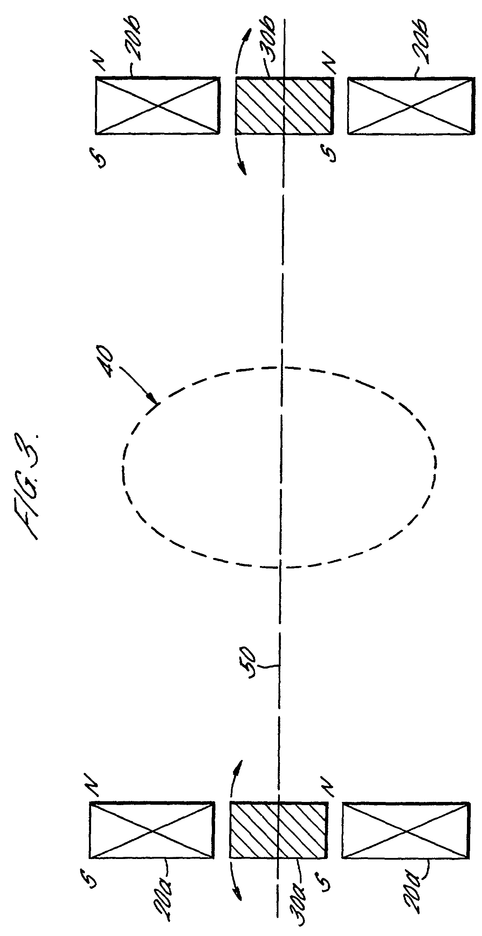

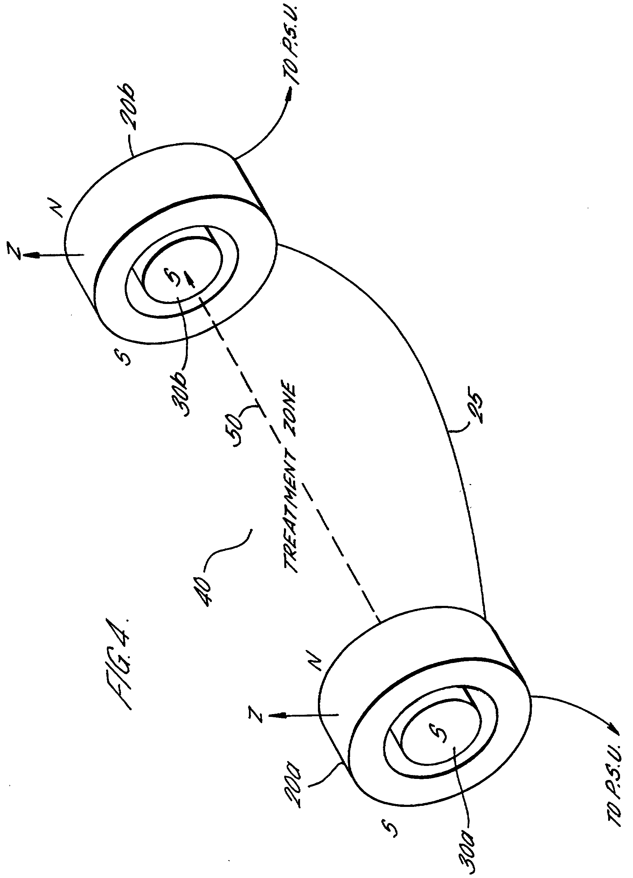

Figure 3 shows a schematic plan view of the electromedical treatment apparatus embodying the present invention. Figure 4 shows the apparatus of Figure 3 in schematic perspective view.

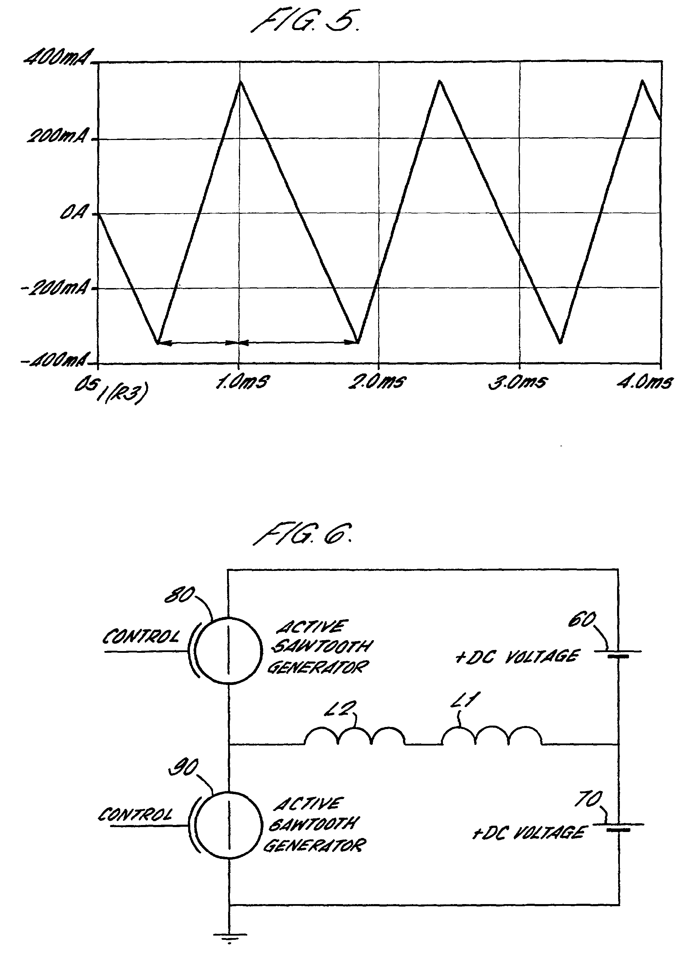

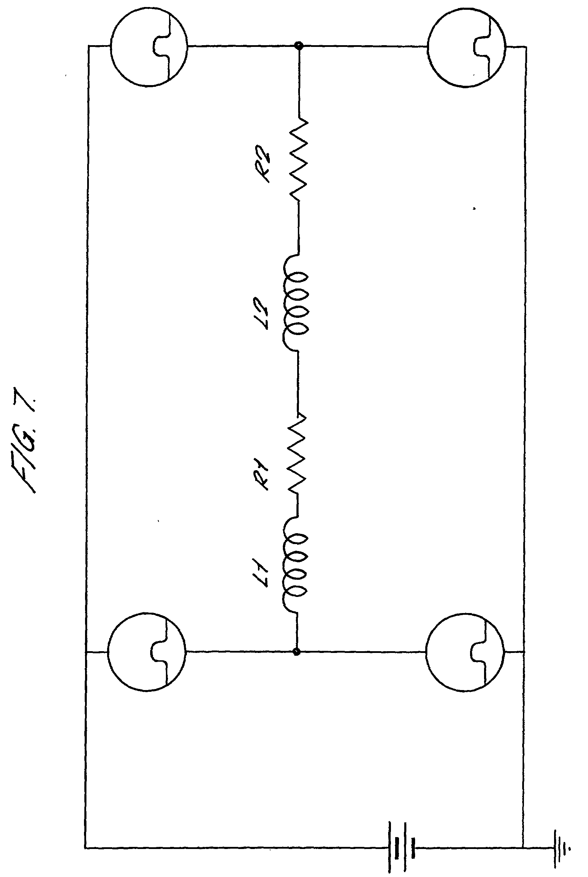

The apparatus comprises a pair of solenoid coils 20a, 20b which are electrically connected together by a wire 25 (Figure 4). Mounted within the bore of each solenoid coil 20a, 20b is a corresponding permanent magnet 30a, 30b. A power supply unit (PSU), not shown in Figures 3 and 4, is connected to each of the solenoid coils 20a, 20b to supply an a.c. signal which in turn causes the solenoid coils to generate a time-varying or dynamic magnetic field. The specific form of the applied a.c. signal will be described in further detail in connection with Figures 5 and 6.

As may be seen from Figures 3 and 4, the coils are arranged, in use, such that the North pole of the first solenoid coil 20a faces the South pole of the second solenoid coil 20b. Likewise, the North pole of the first permanent magnet 30a within the bore of the first solenoid coil 20a faces the South pole of the second permanent magnet 30b within the bore of the second solenoid coil 20b. With this arrangement, the resultant magnetic field generated in the region between two coils, and between the two permanent magnets, is the vector sum of the static magnetic field generated by the two permanent magnets 30a, 30b, and the dynamic magnetic field generated by the two solenoid coils 20a, 20b.

It is important to note that the arrangement shown in Figures 3 and 4 does not contain any magnetic lenses. Nor is any external electric field generated. Thus, the PSU only needs to supply an a.c. signal to the two coils 20a, 20b.

In use, the two coils, with the permanent magnets mounted within their bores, are placed either side of a body part to be treated. For example, to treat a torn ligament in the leg, the first solenoid coil 20a is placed on one side of the ligament, and the second coil 20b is placed on the other side of the ligament such that the two coils face each other and are substantially coaxial, as shown in Figure 3. In this case, the magnetic field lines within a treatment zone 40 where the ligament is to be treated are generally parallel to one another and to the axis of the solenoid coils 20a, 20b.

It has been ascertained that a bias static magnetic field of around 25 µT or more is necessary for the proper operation of the apparatus. Therefore, permanent magnets formed from a ceramics material, such as the "Ferrostrocita-1" from 1DEMAG or the larger "Ferrobarita" from the same manufacturer, may suitably be used. Ceramic magnets have the twin advantages of relatively low cost and very low electrical conductivity. The low electrical conductivity means that eddy currents and thus eddy current losses are insignificant. This in turn allows the permanent magnets to be mounted within the bores of the solenoid coils.

In order to permit the magnetic field strength in the treatment zone 40 to be adjusted, the permanent magnets 30a, 30b are mounted within the bores of the solenoid coils 20a, 20b so as to allow rotation about an axis perpendicular to the common axis 50 of the two solenoid coils. For example, the two permanent magnets may be rotatable about a z-axis as shown in Figure 4, so that the magnets can move in the direction of the arrows shown in Figure 3.

Turning now to Figure 5, the waveform which is preferably applied to the coils of Figures 3 and 4 is shown. It will be seen that the waveform is of sawtooth form in which the rise time of the waveform represents 40% of the period of the wave, and the fall time represents 60% thereof. The frequency is between 100 and 1200 Hz, most typically between 600 and 800 Hz. It will be noted that, in contrast with the waveform that had to be applied to the prior art device of EP-A-0,062,032, the waveform is a true a.c. signal with no d.c. component, that is, it oscillates about a nominal zero amps. It will also be noted that the peak current of the waveform is less than +/-400 mA, with an EMF of, typically, a few Volts at most which can readily be supplied by a small nickel cadmium battery, for example.

There are a number of ways of realizing an electronic circuit capable of producing a suitable sawtooth current. One such circuit is shown in Figure 6, which employ a pair of inductors L1 and L2, two batteries 60, 70, and two active sawtooth generators 80, 90 which typically employ transistor technology to permit adjustment of the ratio between the rise time and fall time of the waveform. In that regard, it is to be understood that although a ratio of 40:60 is preferred, different aspect ratios may be chosen depending upon the particular body part to be treated, and for example, a waveform having the same rise time as fall time may in some cases be desirable.

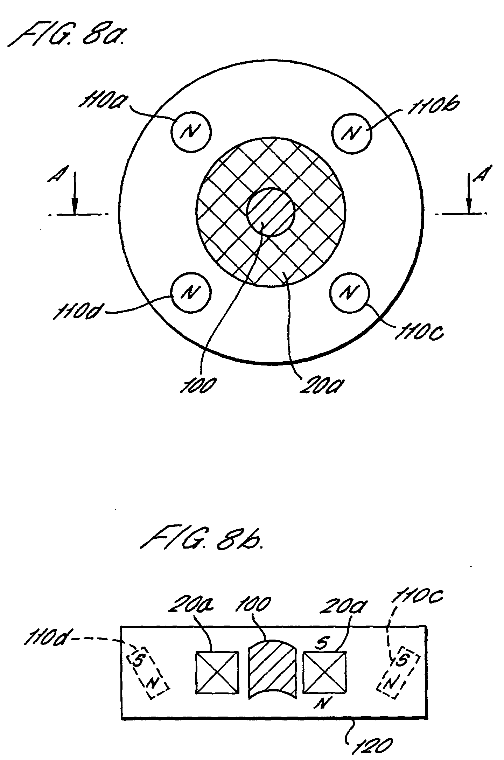

A different circuit for generating a suitable dynamic magnetic field (known as an 'H-bridge' arrangement) is shown in Figure 7. This arrangement requires even less energy to be supplied in order to generate a suitable signal for supply to the coils.

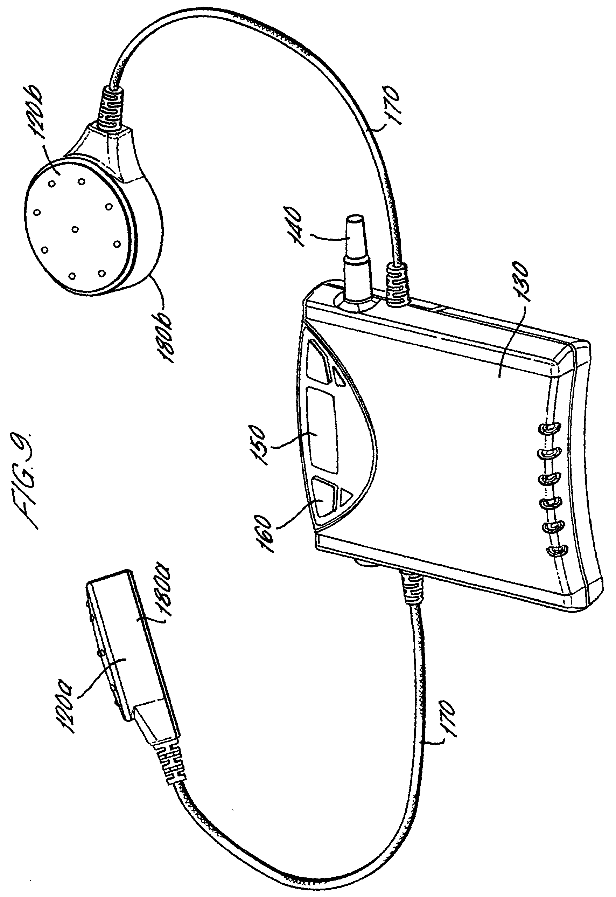

Turning now to Figure 8a and 8b, an alternative embodiment of an electromedical treatment apparatus in accordance with the present invention is shown. Figure 8b shows a sectional view along the line AA' of Figure 8a. Once again, the apparatus comprises a pair of solenoid coils, although in Figures 8a and 8b only one such coil 20a is shown. In contrast to the embodiment of Figures 3 and 4, however, the bore of the solenoid coil 20a contains a soft iron core 100. The purpose of the soft iron core is to enhance further the strength of the dynamic field generated by the solenoid coil 20a. The soft iron core is formed such that the end thereof which faces the treatment region is convex, which focuses the magnetic flux lines towards the body part to be treated. The opposite end of the soft iron core 100 is concave to minimise the spread of the flux lines away from the treated object.

An array of small permanent magnets 110a-110d are mounted within a housing 120 and are arranged proximal to and surrounding the outer diameter of the solenoid coil 20a. As with the embodiment described in connection with Figures 3 and 4, the magnets may be tilted to focus their field away from or towards the treated object, depending upon the desired bias static magnetic field strength at the treatment region.

Turning finally to Figure 9, a schematic perspective view of the apparatus of Figures 3 to 7 is shown mounted in a housing and in combination with a power supply unit. The arrangement of Figure 9 comprises the two solenoid coils 20a, 20b mounted within respective housings 120a and 120b, these housings being suitably formed of a durable plastics material. The permanent magnets 30a, 30b or 110a-110d are also mounted within the housings 120a, 120b, either within the bore of the solenoid coils or surrounding those solenoid coils, as previously described. The arrangement of Figure 9 also includes a power supply unit mounted within a separate power supply unit housing 130, which is also suitably formed of a durable plastics material. Preferably, the power supply unit for generating a suitable waveform for supply to the solenoid coils is as shown in Figure 6 or Figure 7 and does not therefore require an external source of power. This allows the arrangement of Figure 9 to be easily portable. However, to allow charging of the batteries, and to supplement the battery operation, an external mains supply 140 may also be included.

The power supply unit housing 130 includes a liquid crystal display screen 150 to indicate, for example, the state of the battery, the shape and amplitude of the signal being supplied to the solenoid coils and so forth. An array of buttons 160 are also provided to allow adjustment of the signal, to switch the arrangement on and off, and so forth. The solenoid coils 20a and 20b are connected to the power supply unit housing 130 via flexible insulated wires 170.

In use, the region of the body to be treated is identified and then the lower surfaces 180a, 180b of the housings 120a, 120b respectively are placed on either side of that body part. The arrangement of magnet poles is as shown in Figure 3, such that the South pole of both the permanent magnet or magnets and the solenoid coil within the housing 120b faces the North pole of the corresponding magnets in the housing 120a.

It will be apparent from Figure 9 that the apparatus of the present invention, and the ancillary housings for it, do not need to be at all bulky. The presently preferred embodiment uses the magnetic arrangement shown in Figure 3. The outer diameter of the solenoid coils 20a, 20b is approximately 40 mm, and the inner diameter, i.e. the solenoid bore diameter, is approximately 10 mm. The ceramic permanent magnets 30a, 30b inserted into the bore of the solenoid coils 20a, 20b have a typical diameter of 8 mm and a thickness of 8 mm as well. The overall thickness of the solenoid coils 20a, 20b need be no greater than 10 mm. The PSU housing may be held easily in the palm of the hand.

In use, the PSU housing 130 is in preference worn on the arm or leg of the patient, the rear side of the PSU housing 130 and the lower surfaces 180a, 180b of the housings 120a, 120b having Velcro⁽™⁾ fastenings to allow them to be secured to the body. An elastic cuff is placed over the arm or leg, for example, and slid to the treatment location. The Velcro⁽™⁾ patches on the housings 120a, 120b can then be pressed onto the cuff on either side of the body part to be treated. The cuff is preferably large enough to allow the Velcro⁽™⁾ patch on the PSU housing 130 to attach to the cuff as well.

This arrangement, using ceramic permanent magnets, permits the 25 µT minimum static magnetic field to be generated at a distance of approximately 18.6 cm from the centre of the magnet. This allows treatment of even large body parts such as the upper leg.

The power consumption of the arrangement of Figure 9 is typically more than five times less than the power consumption of prior art devices such as shown in the above-identified EP-A-0,062,032.

Whilst a number of embodiments have been described, it will be appreciated that various modifications might be made without departing from the scope of the invention as defined in the following claims. For example, rather than using twin coils/permanent magnets in an axial arrangement with the treatment region "sandwiched" between the two coils, the coils can instead be laid side-by-side. This configuration is particularly suitable for the treatment of back problems where the thickness of the human torso makes the axial arrangement of Figure 4 undesirable. Whilst in this configuration, the minimum composite magnetic field strength occurs closer to the surface of the body, it is often the case that the blood vessels affected in the case of back injuries are in any case closer to the surface of the skin.

Indeed, in one preferred embodiment, a blanket or top-mattress containing a plurality of coils and permanent magnets is envisaged, for placing on or over a bed. Each coil may be individually controllable, and/or each coil/permanent magnet may have individually adjustable orientations. This allows treatment of bed sores, for example, in bed-ridden patients.

## Claims

1. An apparatus for electromedically treating body parts by inducing movement in electrically charged particles in said body part, comprising:
a first coil, to generate a dynamic magnetic field when the coil is supplied with an a.c. signal; and
a first permanent magnet structure to generate a static bias magnetic field, the first permanent magnet structure being arranged in proximity to the first coil such that, in use, a composite magnetic field comprising the dynamic magnetic field superimposed upon the static magnetic field is generated.

2. The apparatus of claim 1, in which the first coil further comprises a soft iron core member, the first coil being arranged at least partially to surround the core member.

3. The apparatus of claim 2, in which the core member has a first surface facing towards a body part to be treated when in use, which first surface is concave.

4. The apparatus of claim 2 or claim 3, in which the or each core member has a second surface facing away from the body part to be treated when in use, which second surface is convex.

5. The apparatus of any preceding claim, in which the first coil is an annular solenoid having a solenoid bore, the first permanent magnet structure being arranged within the solenoid bore of the first coil

6. The apparatus of any one of claims 1 to 4, in which the first coil is an annular solenoid having end faces, the first permanent magnet structure comprising a plurality of permanent magnets arranged radially around the end faces of the first coil.

7. The apparatus of any one of the preceding claims, in which the first permanent magnet structure is formed of a ceramics material.

8. The apparatus of any one of the preceding claims, further comprising a power supply for providing a source of electrical power to the first coil, the power supply comprising a battery having an output and a circuit for generating a time varying signal therefrom.

9. The apparatus of claim 8, in which the circuit is arranged to generate a sawtooth signal from the battery output.

10. The apparatus of claim 9, in which the circuit is arranged to generate a sawtooth signal having a ratio of rise time to fall time between 40:60 and 50:50.

11. The apparatus of claim 8, in which the circuit is arranged to generate a sinusoidal signal from the battery output.

12. The apparatus of claim 1, further comprising a second coil also arranged to generate a dynamic magnetic field when supplied with an a.c. signal, the first and second coils being laterally displaceable from one another; and a second permanent magnet structure arranged in proximity to the second coil, wherein the composite magnetic field is generated in a treatment region defined between the two coils when laterally displaced.

13. The apparatus of claim 12, in which at least one of the first and second coils further comprises a soft iron core member, the or each coil being arranged at least partially to surround the core member.

14. The apparatus of claim 13, in which the or each core member has a first surface facing towards the treatment region in use, which first surface is concave.

15. The apparatus of claim 13 or claim 14, in which the or each core member has a second surface facing away from the treatment region in use, which second surface is convex.

16. The apparatus of any one of claims 12 to 15, in which the first and second coils are each annular solenoids having a solenoid bore, the first permanent magnet structure being arranged within the solenoid bore of the first coil, and the second permanent magnet structure being arranged within the solenoid bore of the second coil.

17. The apparatus of any one of claims 12 to 15, in which the first and second coils are each annular solenoids having end faces, the first and second permanent magnet structures each comprising a plurality of permanent magnets arranged radially around the end faces of each coil.

18. The apparatus of any one of claims 12 to 17, in which the first and second permanent magnet structures are each formed of a ceramics material.

19. The apparatus of any one of claims 12 to 18, further comprising a power supply for providing a source of electrical power to each coil, the power supply comprising a battery having an output and a circuit for generating a time varying signal therefrom.

20. The apparatus of claim 19, in which the circuit is arranged to generate a sawtooth signal from the battery output.

21. The apparatus of claim 20, in which the circuit is arranged to generate a sawtooth signal having a ratio of rise time to fall time between 40:60 and 50:50.

22. The apparatus of claim 19, in which the circuit is arranged to generate a sinusoidal signal from the battery output.

23. The apparatus of any one of claims 11 to 20, in which the orientation of the first and second permanent magnet structures relative to the treatment zone is adjustable so as to permit the magnitude of the static bias magnetic field to be altered.
